# EUROPÄISCHE PATENTSCHRIFT

(11) **EP 1 693 359 B1**
(45) Veröffentlichungstag und Bekanntmachung des Hinweises auf die Patenterteilung: **23.09.2015**
(21) Anmeldenummer: 06002514.5
(22) Anmeldetag: 08.02.2006
(51) Int. Cl.: C07C 67/26, C07C 69/54

(54) **Verfahren zur Herstellung von Hydroxyalkyl(meth)acrylaten**
Process for the preparation of hydroxyalkyl (meth)acrylates
Procédé de préparation de (méth)acrylates d'hydroxyalkyle

(30) Priorität: 22.02.2005 DE 102005008032
(43) Veröffentlichungstag der Anmeldung: 23.08.2006
(73) Patentinhaber: Allnex IP S.à.r.l., 1660 Luxembourg (LU)
(72) Erfinder: Weikard, Jan, Dr., 51519 Odenthal (DE); Gürtler, Christoph, Dr., 50676 Köln (DE)
(74) Vertreter: Destryker, Elise Martine

(56) Entgegenhaltungen:
- EP-A- 0 900 778
- GB-A- 1 480 945
- US-A- 4 069 242
- US-A- 4 126 527
- BECKETT M A ET AL: "A convenient n.m.r. method for the measurement of Lewis acidity at boron centres: correlation of reaction rates of Lewis acid initiated epoxide polymerizations with Lewis acidity" POLYMER, ELSEVIER SCIENCE PUBLISHERS B.V, GB, Bd. 37, Nr. 20, September 1996 (1996-09), Seiten 4629-4631, XP004069466 ISSN: 0032-3861
- DATABASE CA [Online] CHEMICAL ABSTRACTS SERVICE, COLUMBUS, OHIO, US; KIMURA, KAORU ET AL: "Acrylate and methacrylate esters" XP002381405 gefunden im STN Database accession no. 1977:73371 & JP 51 086413 A (TOA GOSEI CHEMICAL INDUSTRY CO., LTD., JAPAN) 29. Juli 1976 (1976-07-29)

## Beschreibung

Die vorliegende Erfindung betrifft ein neues Verfahren zur Herstellung von Hydroxyalkyl(meth)-acrylaten.

(Meth)acrylat bezeichnet dabei im Sinn der vorliegenden Erfindung Ester der Acrylsäure oder der Methacrylsäure oder deren Mischungen. Hydroxyalkyl(meth)acrylate werden unter anderem für die Umsetzung mit isocyanathaltigen Verbindungen zur Herstellung von Urethan(meth)acrylaten, ungesättigten Polyurethandispersionen sowie Dual Cure Härtern für Zwei- oder Mehrkomponentenbeschichtungssysteme verwendet. Die vorgenannten Verbindungsklassen werden insbesondere als Bestandteile von Beschichtungsmitteln verwendet, die durch radikalische Polymerisation härten. Diese kann dabei durch aktinische Strahlung ausgelöst werden. Die Härtung unter Kombination von zwei Reaktionsmechanismen wird vom Fachmann als Dual Cure bezeichnet.

Um besonders hochvernetzte Beschichtungen zu erhalten, ist es zweckmäßig, Verbindungen mit hoher Funktionalität insbesondere an radikalisch polymerisierbaren Doppelbindungen zu verwenden, die sich vorteilhaft durch Verwendung von Hydroxyalkyl(meth)acrylaten hoher Funktionalität an Acrylat- und/oder Methacrylatgruppen herstellen lassen. Um einen gezielten Molekülaufbau und damit u.a. eine niedrige Viskosität zu ermöglichen, ist es weiterhin zweckmäßig, Hydroxyalkyl(meth)acrylate zu verwenden, deren Hydroxyfunktionalität möglichst eng verteilt ist. Daher ist die Herstellung der Hydroxyalkyl(meth)acrylate durch Veresterung von Polyolen wie Trimethylopropan oder Pentaerythrit mit (Meth)acrylsäure als statistisch ablaufender Prozess, der eine breitere Verteilung der Hydroxyfunktionalität erzeugt, nachteilig.

Weiterhin bilden sich häufig Nebenprodukte höheren Molekulargewichts, die z.B. in Analytical Sciences, Nov. 2001, Vol. 17, S. 1295-1299, als Additionsprodukte von Hydroxylgruppen an die C-C-Doppelbindungen von Acrylaten identifiziert wurden. In DE-A 19 860 041 wird 3-Acryloyloxy-2-hydroxypropyl-methacrylat als mögliche Verbindung für eine Reaktion mit Polyisocyanaten zur Herstellung von Dual Cure Härtern offenbart. Details zur Herstellung von 3-Acryloyloxy-2-hydroxypropyl-methacrylat werden jedoch nicht beschrieben.

Die Herstellung von 3-Acryloyloxy-2-hydroxypropyl-methacrylat wird in der Literatur durch Umsetzung von Glycidylmethacrylat mit Acrylsäure, beide in hoher Reinheit kommerziell erhältlich, unter geeigneter Katalyse beschrieben. Angaben zur Reinigung/Reinheit des Produkts werden nicht gemacht. So beschreibt die EP-A 0 900 778 die Reaktion von überschüssiger Acrylsäure nach Veresterungsreaktionen mit Glycidylmethacrylat unter Katalyse von Benzyltriethylammoniumchlorid.

3-Acryloyloxy-2-hydroxypropyl-methacrylat ist weiterhin kommerziell im Feinchemikalienhandel (Fa. Sigma-Aldrich Chemie GmbH, Taufkirchen, DE) erhältlich, die Reinheit nach Gelpermeationsanalyse beträgt weniger als 60 Gew.-%, insbesondere werden unerwünschte höhermolekulare Anteile gefunden. Das Herstellverfahren ist nicht bekannt.

Weiterhin sind aus der Beschichtungstechnologie verschiedenste Katalysatoren für die Reaktion von Glycidylverbindungen mit Carbonsäuren bekannt. Diese Reaktion wird beispielsweise häufig als Vernetzungsreaktion zur Aushärtung von Lacken verwendet. Neben Ammonium- und Phosphoniumsalzen bzw. Aminen und Phosphinen sind weiterhin bestimmte Metallverbindungen beschrieben.

In US 4,069,242 und US 4,126,527 wird die Synthese von Hydroxylacrylaten aus der Umsetzung von Epoxiden mit (Meth)acrylsäure beschrieben. Als Katalysatoren warden Lewis-Säuren in Form von Eisen- und Chrom-Salzen, bzw.von BF₃-Etherat verwendet.

In Polymer 1996, 37(20), 4629-4631 werden bestimmte Eigenschaften von borhaltigen Lewissäuren untersucht. Die Anwendung als Katalysatoren für die Epoxid-Säure-Vernetzung wird erwähnt. Für die Reaktion in Anwesenheit von (Meth) acrylaten kommen allerdings nur Katalysatoren in Frage, die schon bei niedrigen Temperaturen katalytisch aktiv sind, da sonst die Gefahr der ungewollten Polymerisation der (Meth)acrylate besteht. Weiterhin ist für eine chemische Synthese im Vergleich zu einer Vernetzungsreaktion eine größere Selektivität der Katalysatoren erforderlich. Nebenreaktionen wie Michael-analoge Additionen von Hydroxyl- oder Carboxylgruppen an (Meth)acrylate oder Hydroxyl-Epoxid-Reaktionen führen zu unerwünschten weil höhermolekularen Nebenprodukten.

3-Methacryloyloxy-2-hydroxypropyl-methacrylat wird nach destillativer Reinigung mit einer Reinheit größer 85% von der Fa. Röhm GmbH, Darmstadt, DE vertrieben. Die Stabilität des Produkts ist gering, das Produkt muss gekühlt gelagert werden, eine Verwendung im industriellgewerblichen Maßstab ist daher erschwert.

Der vorliegenden Erfindung lag daher die Aufgabe zugrunde, ein Verfahren bereitzustellen, mit dem beispielsweise das 3-Acryloyloxy-2-hydroxypropylmethacrylat ohne aufwendige Reinigung in hoher Reinheit, also ohne einen Gehalt an höhermolekularen oder höher hydroxyfunktionellen Anteilen, einfach und bei tiefen Temperaturen herzustellen ist.

Es wurde gefunden, dass die Umsetzung von beispielsweise Glycidylmethacrylat mit Acrylsäure in Substanz schon bei Temperaturen von 80 °C durch Katalyse mit bestimmten schwach lewissauren Boranverbindungen wie beispielsweise Trisdimethylaminoboran rasch und vollständig abläuft.

Gegenstand der Erfindung ist daher ein Verfahren zur katalysierten Umsetzung von mindestens einer Verbindung A, die mindestens eine Epoxidgruppe und gegebenenfalls eine oder mehrere (Meth)acrylatgruppen aufweist, mit mindestens einer Verbindung B, bei der es sich um Acrylsäure und/oder Methacrylsäure handelt, dadurch gekennzeichnet, dass die Umsetzung in Gegenwart von Tris-(dimethylamino)boran durchgeführt wird.

Ebenfalls beschrieben wird die Verwendung der nach dem erfindungsgemäßen Verfahren hergestellten Verbindungen als Komponente in mit aktinischer Strahlung härtbaren Massen und bei der Synthese von Komponenten für mit aktinischer Strahlung härtbaren Massen.

Als Verbindungen A sind sowohl Monoepoxidverbindungen als auch polyfunktionelle Epoxide, insbesondere di- oder trifunktionelle Epoxide, geeignet. Zu nennen sind beispielsweise epoxidierte Olefine, Glycidylether (cyclo)aliphatischer oder aromatischer Polyole und/oder Glycidylester von gesättigten oder ungesättigten Carbonsäuren. Als Monoepoxidverbindung besonders geeignet sind beispielsweise Glycidylacrylat, Glycidylmethacrylat, Versaticsäureglycidylester, Butylglycidylether, 2-Ethylhexylglycidylether, Phenylglycidylether, o-Kresylglycidylether oder 1,2-Epoxybutan. Bevorzugt ist Glycidylmethacrylat.

Als Polyepoxidverbindungen besonders geeignet sind Polyglycidylverbindungen vom Bisphenol-A- oder Bisphenol-F-Typ sowie deren perhydrierten Derivate oder Glycidylether mehrfunktioneller Alkohole wie Butandiol, Hexandiol, Cyclohexandimethanol, Glycerin, Trimethylolpropan oder Pentaerythrit.

Ebenfalls ist es möglich epoxyfunktionelle Polymerisate von Vinylmonomeren wie z.B. monofunktionelle Acrylate, Methacrylate oder Styrol unter anteiliger Verwendung von z.B. Glycidylmethacrylat einzusetzen.

Bei Verbindung B handelt es sich um Acrylsäure und/oder Methacrylsäure.

Verbindungen A und/oder B enthalten mindestens eine Acrylat- und/oder Methacrylatgruppe. Bevorzugt sind die folgenden Kombinationen Glycidylacrylat und Acrylsäure, Glycidylmethacrylat und Methacrylsäure, Glycidylmethacrylat und Acrylsäure, Glycidylether des Bisphenol A oder des perhydrierten Bisphenol A und Acrylsäure, sowie die Mischungen der genannten Kombinationen. Besonders bevorzugt ist die Kombination von Glycidylmethacrylat und Acrylsäure. Das Equivalentverhältnis von Säure- zu Epoxid kann in weiten Bereichen variiert werden. Bevorzugt wird jedoch ein Equivalentverhältnis von 1,2 zu 1,0 bis 1,0 zu 1,2, insbesondere 1,05 zu 1,00 bis 1,00 zu 1,05. Es kann insbesondere zweckmäßig sein, einen leichten Überschuss an einer Komponente zu verwenden, um zu besonders niedrigen Restgehalten der anderen Komponente im Verfahrensprodukt zu gelangen. Beispielsweise sind mit dem erfindungsgemäßen Verfahren bei geeigneter Wahl der Equivalentverhältnisse Restgehalte an Acrylsäure oder Glycidylmethacrylat unter 0,1 Gew.-% sicher zu realisieren.

Nach dem erfindungsgemäßen Verfahren wird die Umsetzung A mit B durch Tris-(dimethylamino)boran katalysiert.

Der Katalysator kann in beliebigen Mengen eingesetzt werden, man wird aber zweckmäßigerweise die Einsatzmenge auf das erforderliche Minimum beschränken. Die optimale Menge ist naturgemäß abhängig von den jeweils umzusetzenden Stoffen und kann durch entsprechende Versuche ermittelt werden. Üblicherweise wird der Katalysator in Mengen von 0,05 bis 5,0 Gew.-%, bezogen auf die Summe an A und B, eingesetzt. Bevorzugt ist die Verwendung in Mengen von 0,1 bis 1,0 Gew.-%, besonders bevorzugt von 0,1 bis 0,5 Gew.%.

Bevorzugt wird die Reaktion in Anwesenheit von einem oder mehreren Stabilisatoren für Acrylate und Methacrylate durchgeführt. Neben sauerstoffhaltigem Gas eignen sich chemische Stabilisatoren zur Vermeidung vorzeitiger Polymerisation in einer Menge von 0,01 - 1 Gew.-%, bevorzugt 0,1 - 0,5 Gew. -% bezogen auf die Menge der ungesättigten Verbindungen. Solche Stabilisatoren sind beispielsweise in Houben-Weyl, Methoden der organischen Chemie, 4. Auflage, Band XIV/1, Georg Thieme Verlag, Stuttgart 1961, Seite 433 ff. beschrieben. Als Beispiele seien genannt: Kupferverbindungen, dreiwertige Phosphorverbindungen wie Phosphite oder Phosphonite, Natriumdithionit, Natriumhydrogensulfid, Schwefel, Hydrazin, Phenylhydrazin, Hydrazobenzol, N-Phenyl-β-naphthylamin, N-Phenylethanoldiamin, Dinitrobenzol, Picrinsäure, p-Nitrosodimethylanilin, Diphenylnitrosamin, Phenole, z.B. para-Methoxyphenol, 2,5-Di-tert.-Butylhydrochinon, 2,6-Di-tert.-butyl-4-methylphenol, p-tert-Butyl-brenzcatechin oder 2,5-Di-tert.-amyl-hydrochinon, Tetramethyl-thiuramdisulfid, 2-Mercaptobenzthiazol, Dimethyl-dithiocarbaminsäure-natriumsalz, Phenothiazin, N-Oxyl-Verbindungen wie z.B. 2,2,6,6-Tetramethylpiperidin-N-Oxid (TEMPO) oder eines seiner Derivate.

Bevorzugt werden erfindungsgemäß 2,6-Di-tert.-butyl-4-methylphenol, 2,5-Di-tert.-butylhydrochinon und/oder para-Methoxyphenol sowie deren Mischungen eingesetzt.

Die Reaktion kann in Anwesenheit eines gegenüber Edukten und Produkten inerten organischen Lösemittels durchgeführt werden. Beispiele sind Lacklösemittel wie Butylacetat, Solventnaphtha, Methoxypropylacetat oder Kohlenwasserstoffe wie Cyclohexan, Methylcyclohexan oder Isooktan. Nach Ende der Reaktion kann das Lösemittel z.B. destillativ entfernt werden oder im Verfahrensprodukt verbleiben. Bevorzugt wird auf die Verwendung von Lösemittel verzichtet.

Neben Lösemitteln können auch Reaktivverdünner verwendet werden. Solche sind beispielsweise die in der Technologie der Strahlenhärtung von Beschichtungen bekannten Verbindungen (vgl. Römpp Lexikon Chemie, S.491, 10. Aufl. 1998, Georg-Thieme-Verlag, Stuttgart). Beispielhaft seien die Ester der Acrylsäure oder Methacrylsäure erwähnt, bevorzugt der Acrylsäure, der folgenden Alkohole. Einwertige Alkohole sind die isomeren Butanole, Pentanole, Hexanole, Heptanole, Octanole, Nonanole und Decanole, weiterhin cycloaliphatische Alkohole wie Isobornol, Cyclohexanol und alkylierte Cyclohexanole, Dicyclopentanol, arylaliphatische Alkohole wie Phenoxyethanol und Nonylphenylethanol, sowie Tetrahydrofurfurylalkohole.

Weiterhin können alkoxylierte Derivate dieser Alkohole verwendet werden. Zweiwertige Alkohole sind beispielsweise Alkohole wie Ethylenglykol, Propandiol-1,2, Propandiol-1,3, Diethylenglykol, Dipropylenglykol, die isomeren Butandiole, Neopentylglykol, Hexandiol-1,6, 2-Ethylhexandiol und Tripropylenglykol oder auch alkoxylierte Derivate dieser Alkohole. Bevorzugte zweiwertige Alkohole sind Hexandiol-1,6, Dipropylenglykol und Tripropylenglykol. Höherwertige Alkohole sind Glycerin, Trimethylolpropan, Ditrimethylolpropan, Pentaerythrit oder Dipentaerythrit oder deren alkoxylierte Derivate.

Die Umsetzung A mit B erfolgt entweder kontinuierlich z.B. in einem statischem Mischer oder diskontinuierlich z.B. in einem geeigneten Rührgefäß. Bei diskontinuierlicher Fahrweise können die Komponenten in beliebiger Reihenfolge zur Reaktion gebracht werden. Bevorzugt wird eine Komponente vorgelegt, mit der Hauptmenge des Katalysators und mit Stabilisator versetzt und unter Rühren erwärmt. Die andere Komponente wird anschließend auf einmal oder bevorzugt allmählich zudosiert, wobei durch Heizung und Reaktionswärme möglichst eine konstante Temperatur gehalten wird. Durch Analyse wird der Grad des Umsatzes bestimmt. Diese kann spektroskopisch z.B. durch Aufnahme von Infrarot- oder Nahinfrarotspektren erfolgen, es können jedoch auch Analysen z.B. durch Titration, Gaschromatographie oder Bestimmung des Brechungsindex an entnommenen Proben durchgeführt werden. Insbesondere eignet sich sowohl der an titrierten Proben bestimmte Säure- als auch der Epoxidgehalt als Maß für den Umsatz der Reaktion. Die Dosierung und die Reaktion wird bei einer Temperatur zwischen 60 und 130°C, bevorzugt zwischen 65 und 120°C, besonders bevorzugt zwischen 75 und 95°C durchgeführt.

Bei kontinuierlicher Fahrweise können bei genügend kurzer Verweilzeit im Minutenbereich auch Temperaturen oberhalb 130°C verwendet werden.

Bevorzugt wird die Reaktion solange durchgeführt, bis ein Epoxidgehalt unter 0,2 Gew.-%, bevorzugt unter 0,1 Gew.-% (berechnet als M = 42) und eine Säurezahl unter 10 mg KOH/g, besonders bevorzugt unter 5 mg KOH/g erreicht ist. Wird die Reaktion vorher abgebrochen, so kann z.B. durch Anlegen von Vakuum oder Durchleiten eines Gases, welches bevorzugt Sauerstoff enthält, der Restgehalt an flüchtigen Edukten reduziert werden, so dass entsprechend niedrige Epoxid- und Säuregehalte erreicht werden. Es ist ebenfalls möglich, den Epoxidgehalt durch Zugabe von kleinen Mengen an Epoxid-reaktiven Verbindungen wie stärkeren Säuren z.B. Butylphosphat zu senken. Analog können Restgehalte an Säure z.B. durch Reaktion mit Carbodiimiden oder Aziridinen reduziert werden.

Die entstandenen Verfahrensprodukte können sofort weiter umgesetzt oder formuliert werden oder zunächst gelagert oder transportiert werden. Bevorzugt erfolgt der weitere Umsatz ohne weitere Reinigung wie z.B. Extraktion oder Destillation z.B. mit Polyisocyanaten.

Das erfindungsgemäße Verfahren zeichnet sich insbesondere durch eine vergleichsweise hohe Selektivität aus. Werden als A und B jeweils monomere Verbindungen eingesetzt, so beträgt der Gehalt an Bestandteilen im Verfahrensprodukt der nach Gelpermeationschromatographie oligomeren oder polymeren Charakter hat, bevorzugt weniger als 35 besonders bevorzugt weniger als 25 Gew.%.

Die erfindungsgemäßen Verfahrensprodukte eignen sich besonders zur Verwendung zur Herstellung von Rohstoffen und Formulierungen von durch aktinische Strahlung härtbaren Massen. Werden sie als Zwischenprodukte zur Synthese entsprechender Rohstoffe eingesetzt, so kann eine weitere Reaktion beispielsweise mit den im erfindungsgemäßen Verfahren entstandenen und/oder gegebenenfalls weiteren vorhanden Hydroxylgruppen oder durch Addition an vorhandene Doppelbindungen z.B. durch Zugabe primärer und/oder sekundärer Aminogruppen haltiger Verbindungen erfolgen. Insbesondere lassen sich die Hydroxylgruppen durch Urethanisierung und/oder Allophanatisierung nach an sich bekannten Verfahren weiter zur Reaktion bringen. Die erfindungsgemäßen Verfahrensprodukte sowie gegebenenfalls auch die Produkte der weiteren chemischen Umsetzungen eignen sich als Bestandteil von durch aktinische Strahlung härtbaren Massen wie beispielsweise Lacke, Beschichtungen, Druckfarben, Dichtstoffe, Tränkharze und Spachtelmassen.

### Beispiele

Die Säurezahl wird in mg KOH / g Probe angegeben, ihre Bestimmung erfolgt durch Titration mit 0,1 mol/l NaOH-Lösung gegen Bromthymolblau (ethanolische Lösung), Farbumschlag von gelb über grün nach blau, auf Grundlage der DIN 3682.

Die Hydroxylzahl wird in mg KOH / g Probe angegeben, ihre Bestimmung erfolgt durch Titration mit 0,1 mol/l meth. KOH-Lösung nach kalter Acetylierung mit Essigsäureanhydrid unter Katalyse von Dimethylaminopyridin auf der Grundlage der DIN 53240.

Der Epoxidgehalt wird bestimmt, in dem die in Methylenchlorid/Essigsäure gelöste Probe nach Zugabe von Tetrabutylammoniumjodid mit einer Perchlorsäure-Maßlösung (0,1mol/L) titriert wird. Dabei wird das freigewordene Amin sowie vorhandenes basisches Amin erfasst. Das basische (freie) Amin wird analog bestimmt, aber ohne Zusatz von Tetrabutylammoniumjodid. Aus der Differenz ergibt sich der Gehalt an Epoxid in Gew.-%, berechnet als CH-O-CH (MG = 42 g/mol). Die Bestimmung erfolgt auf der Grundlage der DIN 16945.

Gelpermeationschromatographie (GPC): Elutionsmittel THF, RI-Detektion, Integration nach Eichung mit Polystyrolstandards.

Viskositäten: Rotationsviskosimeter, Messungen bei 23°C

Wenn nicht anderweitig vermerkt handelt es sich bei %-Angaben um Gew.-%.

### Beispiel 1: Herstellung eines Epoxyacrylats

In einem beheizbaren Glaskolben mit mechanischem Rührer, Thermometer und Gaseinleitung wurden 142,23 g Epilox® A 19-00 (Bisphenol-A-Epoxidharz der Leuna-Harze GmbH, Leuna, DE mit einem Epoxidequivalentgewicht von 190 g), 0,20 g 2,6-Di-tert.-butyl-4-methylphenol und 0,70 g Tris(dimethyl)aminoboran (Sigma-Aldrich Chemie GmbH, Taufkirchen, DE) vorgelegt und unter Rühren und Gaseinleitung (0,5 1/h Luft) auf 70 °C aufgeheizt. Bei dieser Temperatur wurden 56,67 g Acrylsäure innerhalb von 45 min zudosiert. Im Anschluss wurde bei 90°C gerührt und der Reaktionsverlauf durch Bestimmung der Säurezahl einer entnommenen Probe überprüft. Nach 4 h betrug die Säurezahl 34 mg KOH/g, nach 12 h 8 mg KOH/g und nach 15 h 5 mg KOH/g. Anschließend wurde mit 50,00 g Laromer^{®} HDDA (Hexandioldiacrylat, BASF AG, Ludwigshafen, DE) verdünnt und abgekühlt. Viskosität 14700 mPas, Farbzahl 48 APHA, Säurezahl 5,0 mg KOH/g.

### Umsetzung von Glycidylmethacrylat mit Acrylsäure:

Jeweils 13,27 g Glycidylmethacrylat, 0,02 g 2,6-Di-tert.-butyl-4-methylphenol, 6,78 g Acrylsäure und je 0,10 g Katalysator gemäß Tabelle 1 wurden in einem Glasgefäß mit kleiner Öffnung unter magnetischem Rühren bei 80°C umgesetzt. Nach 24 und 48 Stunden wurde die Säurezahl bestimmt. War diese nach 48 h größer als 4 mg KOH/g wurde der Ansatz ohne weitere Analytik verworfen.

**Tabelle 1: Versuchsserie 80°C**

| Bsp. | Katalysator | Säurezahl nach 24 h (mg KOH/g) | Säurezahl nach 48 h (mg KOH/g) | Hydroxylzahl (mg KOH/g) | GPC Hauptsignal (Flächen-%) | Bemerkung |
|---|---|---|---|---|---|---|
| V2 | Triethylamin | 12,6 | 1,8 | 220 | 68 | Gelb |
| V3 | Diazabicyclooctan | 15,5 | 5,9 | 232 | 80 | Gelb |
| V4 | Zinn(II)octoat | Innerhalb 24 h geliert | | | | |
| V5 | Dibutylzinndilaurat | 22,0 | 11,2 | - | - | - |
| V6 | Thiodiglycol | 14,2 | 8,3 | - | - | - |
| V7 | Triethylbenzylammoniumchlorid | 9,2 | 2,1 | 243 | 79 | - |
| V8 | Triphenylphosphin | 6,6 | 1,5 | 220 | 75 | - |
| 9 | Tris(dimethylamino)-boran | 4,0 | 1,6 | 238 | 80 | - |

Die Katalysatoren der Vergleichsbeispiele V2 bis V6 zeigen eine massenbezogen deutlich geringere, die von V7 und V8 eine geringere Aktivität als der Katalysator im erfindungsgemäßen Beispiel 9. Das Reaktionsprodukt ist nach Säurezahl, Hydroxylzahl und Reinheit (GPC-Analyse) den Vergleichsprodukten mindestens ebenbürtig.

## Patentansprüche

1. Verfahren zur Umsetzung von mindestens einer Verbindung A, die mindestens eine Epoxidgruppe und gegebenenfalls eine oder mehrere (Meth)acrylatgruppen aufweist, mit mindestens einer Verbindung B, bei der es sich um Acrylsäure und/oder Methacrylsäure handelt, **dadurch gekennzeichnet, dass** die Umsetzung in Gegenwart von Tris-(dimethylamino)boran durchgeführt wird.

2. Verfahren gemäß Anspruch 1, **dadurch gekennzeichnet, dass** 0,05 bis 5,0 Gew.-%, bezogen auf die Summe von A und B, an Tris-(dimethylamino)boran eingesetzt werden.

3. Verfahren gemäß Anspruch 1, **dadurch gekennzeichnet, dass** 0,1 bis 1,0 Gew.-%, bezogen auf die Summe von A und B, an Tris-(dimethylamino)boran eingesetzt werden.

4. Verfahren gemäß Anspruch 1, **dadurch gekennzeichnet, dass** 0,1 bis 0,5 Gew.-%, bezogen auf die Summe von A und B, an Tris-(dimethylamino)boran eingesetzt werden.

5. Verfahren nach Ansprüchen 1 bis 4, **dadurch gekennzeichnet, dass** es sich bei Verbindung A um Glycidylmethacrylat und/oder Glycidylacrylat handelt.

6. Verfahren nach Ansprüchen 1 bis 5, **dadurch gekennzeichnet, dass** es sich bei Verbindung A um Glycidylmethacrylat und bei Verbindung B um Acrylsäure handelt.

## Claims

1. Process for reacting at least one compound A having at least one epoxide group and optionally one or more (meth)acrylate groups with at least one compound B, which is acrylic acid and/or methacrylic acid, **characterized in that** the reaction is conducted in the presence of tris(dimethylamino)borane.

2. Process according to Claim 1, **characterized in that** 0.05 to 5.0% by weight of tris(dimethylamino)borane is used, based on the sum total of A and B.

3. Process according to Claim 1, **characterized in that** 0.1 to 1.0% by weight of tris(dimethylamino)borane is used, based on the sum total of A and B.

4. Process according to Claim 1, **characterized in that** 0.1 to 0.5% by weight of tris(dimethylamino)borane is used, based on the sum total of A and B.

5. Process according to Claims 1 to 4, **characterized in that** compound A is glycidyl methacrylate and/or glycidyl acrylate.

6. Process according to Claims 1 to 5, **characterized in that** compound A is glycidyl methacrylate and compound B is acrylic acid.

## Revendications

1. Procédé de mise en réaction d'au moins un composé A, qui comprend au moins un groupe époxyde et éventuellement un ou plusieurs groupes (méth)acrylate, avec au moins un composé B, qui est l'acide acrylique et/ou l'acide méthacrylique, **caractérisé en ce que** la mise en réaction est réalisé en présence de tris(diméthylamino)borane.

2. Procédé selon la revendication 1, **caractérisé en ce que** 0,05 à 5,0 % en poids, par rapport à la somme de A et B, de tris(diméthylamino)borane est utilisé.

3. Procédé selon la revendication 1, **caractérisé en ce que** 0,1 à 1,0 % en poids, par rapport à la somme de A et B, de tris(diméthylamino)borane est utilisé.

4. Procédé selon la revendication 1, **caractérisé en ce que** 0,1 à 0,5 % en poids, par rapport à la somme de A et B, de tris(diméthylamino)borane est utilisé.

5. Procédé selon les revendications 1 à 4, **caractérisé en ce que** le composé A est le méthacrylate de glycidyle et/ou l'acrylate de glycidyle.

6. Procédé selon les revendications 1 à 5, **caractérisé en ce que** le composé A est le méthacrylate de glycidyle et le composé B est l'acide acrylique.
